# EUROPÄISCHE PATENTSCHRIFT

(11) **EP 0 829 230 B1**
(45) Veröffentlichungstag und Bekanntmachung des Hinweises auf die Patenterteilung: **20.10.2004**
(21) Anmeldenummer: 97810601.1
(22) Anmeldetag: 26.08.1997
(51) Int. Cl.: A61B 5/085, F16D 3/02

(54) **Vorrichtung und Verfahren zum Messen des Atemwegwiderstandes**
Device and method for measuring the airway impedance
Dispositif et procédé destinés à la mesure de l'impédance des voies respiratoires

(30) Priorität: 13.09.1996 CH 224796; 24.10.1996 CH 260896
(43) Veröffentlichungstag der Anmeldung: 18.03.1998
(73) Patentinhaber: Schiller AG, 6340 Baar (CH)
(72) Erfinder: Schiller, Alfred, 8914 Aeugst a.A. (CH)
(74) Vertreter: Hepp, Dieter

(56) Entgegenhaltungen:
- EP-A- 0 419 113
- DE-A- 3 608 566

## Beschreibung

Die Erfindung betrifft eine Vorrichtung und ein Verfahren zum Messen des Atemwegwiderstandes gemäss dem Oberbegriff der unabhängigen Patentansprüche.

Zur Früherkennung von Erkrankungen der Atemwege von Personen ist es bekannt, den Fluss der ausgestossenen Atmungsluft zu bestimmen. Zusätzliche Diagnosemöglichkeiten ergeben sich, wenn der innere Widerstand der Atemwege gemessen wird. Für solche Messungen sind sogenannte Unterbrecher- oder Shutter-Messmethoden bekannt. Bei diesen Methoden stösst der Proband die Atemluft durch einen Luftdurchlass in einem Messgerät mit einer Einlass- und einer Auslassöffnung. In einem ersten Schritt wird der Fluss der ausgestossenen Atemluft durch den Durchlass gemessen. In einem zweiten Schritt wird der Druck, der sich im Inneren der Atemwege aufbaut gemessen. Dazu wird der Durchlass in dem Messgerät kurzzeitig geschlossen, so dass sich in den Atemwegen und im mundseitigen Teil des Messgerätes ein Druck aufbaut, der dem inneren Druck in den Atemwegen entspricht. Das Verschliessen des Durchlasses erfolgt während einer kurzen Zeitspanne, beispielsweise während 30 bis 100 Millisekunden. Die Kenntnis des Flusses und des aufgebauten Drucks erlaubt das Bestimmen des inneren Widerstandes der Atemwege. Der Widerstand wird gebildet durch den Quotient aus innerem Druck der Atemwege und Fluss der ausgestossenen Atemluft. Aus dem gemessenen Fluss und dem ermittelten Druck kann dadurch der innere Widerstand der Atemwege bestimmt werden.

Der Luftdurchlass des Messgerätes kann auch in Intervallen während eines Ausatmungsvorgangs wiederholt geschlossen werden. Dadurch lässt sich ein Profil des Atemwegwiderstandes in Abhängigkeit vom Lungeninhalt erzeugen.

Aus der EP 419 113 A1 ist beispielsweise eine Vorrichtung zum Messen des Atemwegwiderstandes bekannt, bei welcher eine Bohrung in einem Gehäuse durch eine elliptische Verschlussklappe temporär verschlossen werden kann. Ein ähnliches Messprinzip ist aus der DE OS 24 13 960 oder aus der DE 35 17 786 A1 bekannt.

Der Durchlass des Messgerätes muss dabei in sehr kurzer Zeit, vorzugsweise im Bereich von einigen Millisekunden verschlossen werden. Daraus ergeben sich hohe Beschleunigungswerte für die Verschlussklappe, was grosses Drehmoment für den Antrieb bedingt. Bei den bekannten Vorrichtungen sind demnach verhältnismässig leistungsstarke und damit auch grosse und teure Antriebsmotoren erforderlich. Dies verteuert die Messgeräte, erhöht den Stromverbrauch und führt zu grossen Geräten. Bei den bekannten Vorrichtungen ergeben sich auch Probleme beim Reinigen der Messvorrichtung. Da das Antriebsorgan und die Verschlussklappe fest miteinander verbunden sind, kann der Motor nur schlecht von dem übrigen Teil der Vorrichtung getrennt werden. Die Messvorrichtung kann deshalb nicht einfach gespült oder desinfiziert werden. Auch Wegwerfkomponenten können nicht eingesetzt werden. Reinigung und Einsatz steriler Komponenten ist aber bei diesem Anwendungsbereich unerlässlich.

Die vorliegende Erfindung stellt sich daher zur Aufgabe, die Nachteile des Bekannten zu vermeiden, insbesondere eine Vorrichtung zum Messen des Atemwegwiderstandes zu schaffen, welche ökonomisch, einfach und kompakt herstellbar ist, die kürzeste Verschliess- und Öffnungzeiten garantiert und überdies ein leichtes Austauschen oder Reinigen des Flussensors ermöglicht. Insbesondere soll auch eine Vorrichtung geschaffen werden, die dabei mit kleineren Antriebsmotoren zuverlässig betreibbar ist. Eine weitere Aufgabe der Erfindung besteht darin, ein Verfahren zum Verschliessen des Luftdurchlasses einer Vorrichtung zum Messen des Atemwegwiderstandes zu schaffen, welches mit im Vergleich zu Motoren von herkömmlichen Vorrichtungen kompakteren und leistungsschwächeren Motoren ausführbar ist. Erfindungsgemäss werden diese Aufgaben mit einer Vorrichtung und einem Verfahren mit den Merkmalen des kennzeichnenden Teils der unabhängigen Patentansprüche gelöst.

Eine Vorrichtung zum Messen des Atemwegwiderstandes besteht im wesentlichen aus einem Gehäuse, mit einem Luftdurchlass mit einer Einlass- und einer Auslassöffnung. Bei der Messung des Atemwegwiderstandes stösst der Proband die Atemluft durch den Luftdurchlass aus. Die Vorrichtung enthält Mittel zum Messen des Flusses der ausgestossenen Atemluft durch den Durchlass sowie eine Verschliessanordnung zum temporären Verschliessen des Luftdurchlasses. Die Mittel zum Messen des Flusses können beispielsweise aus einem in dem Luftdurchlass angeordneten Strömungswiderstand mit einer Druckmessanordnung vor und nach dem Widerstand bestehen. Aufgrund des Druckabfalls am Widerstand wird dabei der Fluss der ausgestossenen Atemluft bestimmt.

Das Gehäuse weist ausserdem eine Messanordnung zum Messen des Druckes zwischen der Einlassöffnung und der Verschliessanordnung auf.

Die Verschliessanordnung ist durch Antriebsmittel zwischen einer Ausgangsposition und einer Verschlussposition bewegbar. In der Ausgangsposition ist der Luftdurchlass im wesentlichen unverschlossen, in der Verschlussposition ist der Luftdurchlass durch die Verschliessanordnung dichtend verschlossen, so dass sich zwischen der Verschliessanordnung und der Einlassöffnung der Vorrichtung ein Druck aufbauen kann.

Die Verschliessanordnung und die Antriebsmittel sind ausserdem über eine Kupplungsanordnung miteinander verbindbar, so dass der Luftdurchlass durch Betätigen der Antriebsmittel verschliessbar ist. Die Antriebsmittel sind so ausgelegt oder angeordnet, dass sie lastfrei von einer Ruheposition in einen Wirkzustand beschleunigbar oder bewegbar sind. In dem Wirkzustand haben die Antriebsmittel einen vorbestimmten Minimalenergieinhalt erreicht. Dieser kann durch eine bestimmte Wirkgeschwindigkeit oder Drehzahl, aber auch eine potentielle Energie (Federspannung) definiert sein. Unter lastfrei wird in diesem Zusammenhang verstanden, dass die Antriebsmittel ohne die äussere Last der Verschliesseinrichtung anlaufen. Dies bedeutet, dass im Zeitpunkt des Anlaufens keine Kopplung zwischen Antriebsmitteln und Verschliessanordnung besteht. Erst im Wirkzustand treten die Antriebsmittel mit der Verschliessanordnung in Eingriff.

Wesentlich ist dabei, dass die Antriebsmittel zu dem Zeitpunkt, in dem sie in Verbindung mit der Verschliessanordnung treten, bereits einen relativ hohen Energieinhalt (Rotationsenergie, Bewegungsenergie, Federspannung) aufweisen. Der Energieinhalt (beispielsweise eine Rotationsenergie) wird aufgebaut, während die Verschliessanordnung immer noch geschlossen bleibt. Diese Anordnung erlaubt die Verwendung von verhältnismässig kleinen oder leistungsschwachen Antriebsmitteln, beispielsweise kleinen Elektromotoren. Die verschliessanordnung steht still, während die Antriebsmittel von einer Ruheposition auf eine gewisse Geschwindigkeit beschleunigen oder sich in einen Zustand begeben. Erst nach Erreichen dieser Geschwindigkeit oder des Zustands (und der dabei gespeicherten Energie) treten die Antriebsmittel mit der Verschliessanordnung in Eingriff.

Durch die Summe aus kinetischer und/oder Rotationsenergie und die Antriebsenergie des Antriebsmittels, wird die Verschliessanordnung beschleunigt und in kürzester Zeit von der Ausgangsposition in eine Verschlussposition (oder von der Verschlussposition wieder zurück in die Ausgangsposition) bewegt. Weil die Antriebsmittel und die Verschliessanordnung nicht starr miteinander verbunden sind, sind die Antriebsmittel auch rasch und einfach von Rest der Messvorrichtung zu lösen. Auf diese Weise können das Gehäuse mit dem Luftdurchlass und der Verschliessanordnung einfach gereinigt oder als Wegwerfkomponente ausgelegt werden.

In einem besonders vorteilhaften Ausführungsbeispiel ist der Durchlass des Gehäuses rund ausgebildet und die Verschliessanordnung besteht aus einer drehbar gelagerten Verschlussklappe. Besonders günstig ist es, wenn die Antriebsmittel aus einem Elektromotor bestehen, dessen Achse konzentrisch zur Drehachse der Verschlussklappe angeordnet ist.

Der Motor kann mit einem Mitnehmerstift versehen sein, der mit einer exzentrischen Anschlagfläche der Verschlussklappe in Eingriff bringbar ist. Beim Betätigen der Antriebsmittel dreht sich der Motor so lange frei, bis der Mitnehmerstift auf die Anschlagfläche der Verschlussklappe trifft. In diesem Zeitpunkt bewegt sich die Verschlussklappe aus ihrer Ausgangsposition weg. Vorteilhaft ist dabei, dass der Motor im Zeitpunkt, in dem der Mitnehmerstift auf die Anschlagfläche trifft, bereits eine bestimmte Rotations-Energie aufgebaut hat, die der Verschlussklappe weitergegeben werden kann. Die Verschlussklappe wird danach vom Motor in die gewünschte neue Position gedreht. Vorzugsweise wird diese Position durch einen Anschlag im Inneren des Luftdurchlasses der Messvorrichtung definiert. Im vorliegenden Ausführungsbeispiel, bei welchem die Verschlussklappe von einer Ausgangsposition in eine Verschlussposition (und zurück) bewegt wird, sind vorzugsweise zwei Anschläge, d.h. je ein Anschlag pro Position angeordnet. Es ist aber auch denkbar, eine elliptische Verschlussklappe in einer runden Öffnung einzusetzen. Auf diese Weise lässt sich der die Verschlussposition definierende Anschlag vermeiden.

Selbstverständlich ist eine Vielzahl von anderen Kopplungen zwischen Antriebsmitteln und der Verschliessanordnung möglich. Denkbar sind beispielsweise Fliehkraftkupplungen, Zahnräder, welche nur auf einem bestimmten Winkelbereich mit Zähnen versehen sind oder Federn (Spiral- oder Schraubenfedern), welche in einen Wirkzustand mit einer gewünschten potentiellen Energie gebracht werden. Weiter ist es denkbar, lineare anstelle von sich drehenden Antriebsmitteln zu verwenden.

Das erfindungsgemässe Verfahren zum temporären Verschliessen und Öffnen des Luftdurchlasses einer Vorrichtung zum Messen des Atemwegwiderstandes kennzeichnet sich im wesentlichen dadurch aus, dass in einem ersten Schritt Antriebsmittel zum Bewegen einer Verschliessanordnung lastfrei von einer Ruheposition in einem Wirkzustand beschleunigt oder bewegt werden. Im Wirkzustand weisen die Antriebsmittel eine kinetische und/oder Rotations-Energie oder eine potentielle Energie auf, die einen bestimmten Wert hat. Erst im Wirkzustand werden die Antriebsmittel mit einer Verschliessanordnung zum Verschliessen des Luftdurchlasses in Eingriff gebracht. Anschliessend wird die Verschliessanordnung durch die Antriebsmittel von einer Ausgangsposition in eine Verschlussposition bewegt.

Der Grundgedanke der vorliegenden Erfindung liegt also im wesentlichen darin, dass einerseits die Antriebsmittel nicht starr mit der Verschliessanordnung verbunden sind, und dass andererseits die Antriebsmittel eine bestimmte Energie aufbauen können, bevor sie überhaupt in Eingriff mit der zu beschleunigenden Masse der Verschliessanordnung treten.

Anstelle einer drehbar gelagerten Verschlussklappe sind auch Verschliessanordnungen mit einem anderen Aufbau denkbar. So können beispielsweise rechteckige oder in lamellenform aufgebaute Verschliessanordnungen verwendet werden. Lamellenförmige Verschliessanordnungen zeichnen sich durch einen im Vergleich zu einer einstückig ausgebildeten Verschlussklappe reduzierten Platzbedarf aus.

Die Erfindung wird im folgenden anhand der Zeichnungen und in Ausführungsbeispielen genauer erläutert. Es zeigen:
- Figur 1: eine perspektivische Darstellung einer aufgeschnittenen, erfindungsgemässen Vorrichtung,
- Figur 2: einen Querschnitt durch eine erfindungsgemässe Vorrichtung,
- Figuren 3a bis 3e: eine Draufsicht auf die erfindungsgemässe Vorrichtung in verschiedenen Arbeitspositionen,
- Figur 4: einen Querschnitt eines weiteren Ausführungsbeispiels einer erfindungsgemässen Vorrichtung,
- Figuren 5a und 5b: ein weiteres Ausführungsbeispiel einer Verschliessanordnung einer erfindungsgemässen Vorrichtung in geöffnetem Zustand (Figur 5b) und in geschlossenem Zustand (Figur 5a), und
- Figur 6: eine schematische Darstellung der erfindungsgemässen Vorrichtung in einer allgemeinen Form.

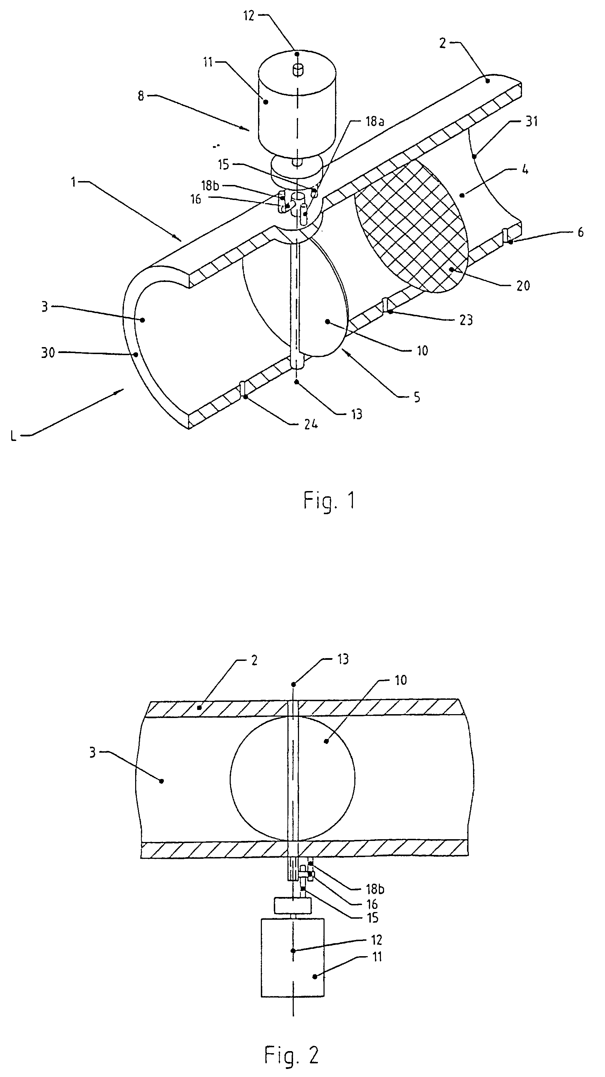

Figur 1 zeigt eine schematische, perspektivische Darstellung einer aufgeschnittenen, erfindungsgemässen Messvorrichtung 1. Die Messvorrichtung 1 besteht im wesentlichen aus einem Gehäuse 2, welches einen Luftdurchlass 3 mit einer Einlassöffnung 30 und einer Auslassöffnung 31 aufweist. Zum Bestimmen des Atemwegwiderstandes eines Probanden stösst der Proband die Atemluft L durch die Einlassöffnung 30 der Messvorrichtung 1. Die Vorrichtung 1 weist ausserdem Messmittel 4 zum Messen des Flusses der ausgestossenen Atemluft auf. Die Messmittel 4 bestehen im wesentlichen aus einem Strömungswiderstand 20, beispielsweise in der Form eines Siebes und aus zwei Druckmessanordnungen 6, 23, welche den Druck auf beiden Seiten des Strömungswiderstandes 20 messen.

Zum Bestimmen des Atemwegwiderstandes muss zusätzlich zum Fluss der ausgestossenen Atemluft L auch der Druck gemessen werden, der sich im Inneren der Atemwege aufbaut. Dazu wird die sogenannte Unterbrechermethode angewandt. Der Luftdurchlass 3 wird mittels einer Verschliessanordnung 5 temporär verschlossen. Figur 1 zeigt die Messvorrichtung 1 in verschlossenem Zustand. Der Luftdurchlass wird während 30 bis 100 Millisekunden, typischerweise 70 Millisekunden verschlossen. Wenn die Verschliessanordnung 5 den Durchlass 3 abgeschlossen hat, wird mittels einem Druckmesser 24 der Druck gemessen, der sich im Inneren der Messvorrichtung 1 aufbaut. Dieser Druck entspricht dem inneren Druck in den Atemwegen.

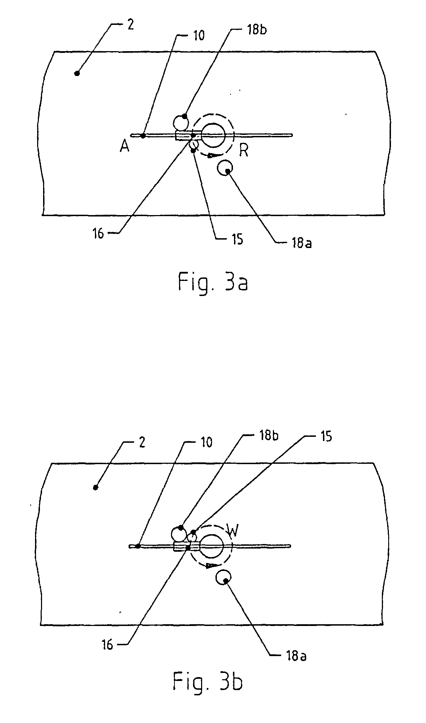

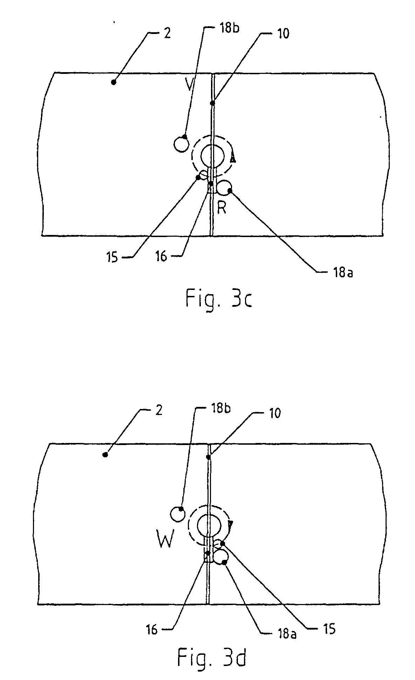

Im Ausführungsbeispiel gemäss Figur 1 besteht die Verschliessanordnung 5 aus einer Verschlussklappe 10, welche über Antriebsmittel 8 zwischen einer Ausgangsposition A (Fig. 3a), in welcher der Luftdurchlass 3 nicht verschlossen ist, in eine Verschlussposition V (Fig. 3c) bewegt werden kann. Die Antriebsmittel 8 bestehen aus einem Elektromotor 11, der mit der Verschlussklappe 10 verbindbar ist. Auf der Innenoder Aussenseite des Luftdurchlasses 3 sind ausserdem zwei Anschläge 18a, 18b vorgesehen, welche die beiden Endpositionen der Verschlussklappe 10 (Ausgangsposition A, Verschlussposition V) definieren. Die Verschlussklappe 10 wird also durch die Antriebsmittel 8 zwischen den beiden durch die Anschläge 18a, 18b definierten Positionen bewegt. Die Bewegung dauert im allgemeinen einige Millisekunden.

Die Verschlussklappe 10 ist nicht starr mit dem Motor 11 verbunden. Dies erlaubt ein rasches Trennen von Motor 11, respektiv der gesamten Antriebsmittel 8 und dem Gehäuse 2 der Messvorrichtung 1. Das ermöglicht ein einfaches Reinigen des Gehäuses oder die Verwendung von wegwerfbaren Gehäusen.

Die Verschlussklappe 10 ist drehbar um eine Achse 13 im Gehäuse 2 gelagert. Die Achse 12 des Motors 11 ist koaxial zur Drehachse 13 der Verschlussklappe 10 angeordnet. Der Motor weist einen Mitnehmerstift 15 auf, der mit einer Anschlagfläche 16 der Verschlussklappe in Eingriff gebracht werden kann. Zum Bewegen der Verschlussklappe 10 von der Ausgangsposition A in die Verschlussposition V (oder zurück) wird der Motor 11 betätigt. Weil der Motor 11 nicht starr mit der Verschlussklappe 10 verbunden ist, beschleunigt er in einem ersten Schritt weitgehend lastfrei, während die Verschlussklappe 10 in ihrer Position verbleibt. Unter lastfrei wird in diesem Zusammenhang verstanden, dass der Motor nur gegen seine eigene Trägheit, nicht aber mit der externen Last der Verschlussklappe 10 anläuft. Nachdem der Motor eine Drehung von annähernd 360 Grad durchgeführt hat (siehe Figuren 3a bis 3e), tritt der Mitnehmerstift 15 mit der Anschlagfläche 16 in Eingriff. Dadurch wird die Verschlussklappe 10 von der Ausgangsposition A in die Verschlussposition V (oder umgekehrt) gebracht. Die Anschläge 18a, 18b definieren die Endpositionen dieser Bewegung.

Weil der Motor in dem Zeitpunkt, in dem der Mitnehmerstift 15 mit der Anschlagfläche 16 in Eingriff tritt, bereits Rotations-Energie aufgebaut hat, wird die Verschlussklappe 10 sehr schnell beschleunigt. Dies erlaubt den Einsatz von vergleichsweise leistungsschwachen Motoren 11 ohne dass lange Verschluss- oder Öffnungszeiten in Kauf genommen werden müssten.

Figur 2 zeigt einen Querschnitt durch eine erfindungsgemässe Vorrichtung, bei welcher die Verschlussklappe 10 in der Ausgangsposition A steht, in der der Durchlass 3 nicht verschlossen ist. Die Ausgangsposition A wird durch den Anschlag 18b im Inneren des Luftdurchlasses 3 definiert. Figur 2 zeigt, wie der Motor 11 und die Verschlussklappe 10 bezüglich ihrer Drehachsen 12, 13 koaxial angeordnet sind. Die Verschlussklappe 10 und der Motor 11 sind so angeordnet, dass sie über einen gewissen Winkelumfang frei drehbar gelagert sind. Spätestens nach einer Drehung von 360 Grad tritt der Motor 11 über den Mitnehmerstift 15 mit der Anschlagfläche 16 der Verschlussklappe 10 in Eingriff.

Figuren 3a bis 3e illustrieren den Öffnungs- und Verschlussvorgang.

Figur 3a zeigt schematisch die Draufsicht auf eine erfindungsgemässe Vorrichtung, bei welcher die Verschlussklappe 10 in der Ausgangsposition A steht. In diesem Zustand wird mit der Vorrichtung der Fluss der ausgestossenen Atemluft L gemessen. Die Position der Verschlussklappe 10 wird durch den Anschlag 18b definiert. Gemäss Figur 3a wird die Verschlussklappe 10 durch den Mitnehmerstift 15 an den Anschlag 18b gepresst. Der Motor 11 befindet sich in einer Ruheposition R. Dies ist nicht zwingend, hilft aber, die Ausgangsposition A der Verschlussklappe 10 genau zu definieren und die Verschlussklappe 10 bis zum Betätigen des Motors in dieser Position zu halten. Wenn der Luftdurchlass 3 mit der Verschlussklappe 10 verschlossen werden soll, wird der Motor 11 gemäss Ansicht in Figur 3a im Uhrzeigersinn in Bewegung gesetzt. Der Motor kann dadurch über einen Winkelbereich von annähernd 360 Grad frei beschleunigt werden, bis er in einem Wirkzustand W (siehe Figur 3b) in Eingriff mit der Anschlagfläche 16 tritt. In diesem Zeitpunkt hat der Motor 11 bereits genügend Rotations-Energie aufgebaut, um die Verschlussklappe 10 rasch zu beschleunigen.

Figur 3c zeigt die erfindungsgemässe Vorrichtung 1, bei welcher die Verschlussklappe 10 sich in der Verschlussposition V befindet. Die Verschlussklappe 10 liegt am Anschlage 18a an. Der Mitnehmerstift 15 steht immer noch im Eingriff mit der Anschlagfläche 16. Dadurch wird die Verschlussklappe 10 auch in der Verschlussposition V genau definiert festgehalten.

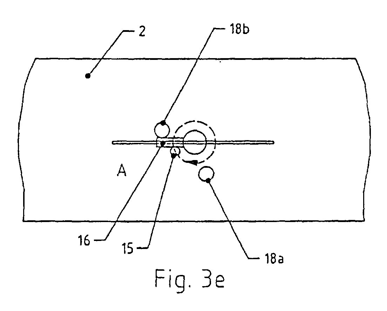

Zum Zurückstellen der Verschlussklappe 10 in die Ausgangsposition A wird der Motor 11 in umgekehrter Richtung (in der Ansicht gemäss Figur 3d im Gegenuhrzeigersinn) betätigt. Der Motor 11 kann erneut über einen Winkelumfang von annähernd 360 Grad lastfrei beschleunigen. Nach einer Drehung von 360 Grad tritt der Mitnehmerstift 15 in einem Wirkzustand W mit der Anschlagfläche 16 der Verschlussklappe 10 in Eingriff. Die Verschlussklappe wird dadurch in die Ausgangsposition A (siehe Figur 3e) gebracht und gegen den ersten Anschlag 18 gedrückt.

Figur 3e entspricht im wesentlichen Figur 3a, wobei in Figur 3e deutlich wird, dass der Mitnehmerstift 15 die Verschlussklappe 10 im allgemeinen gegen den Anschlag 18b drückt, da die Bewegung der Verschlussklappe 10 erst durch den Anschlag 18b gestoppt wird.

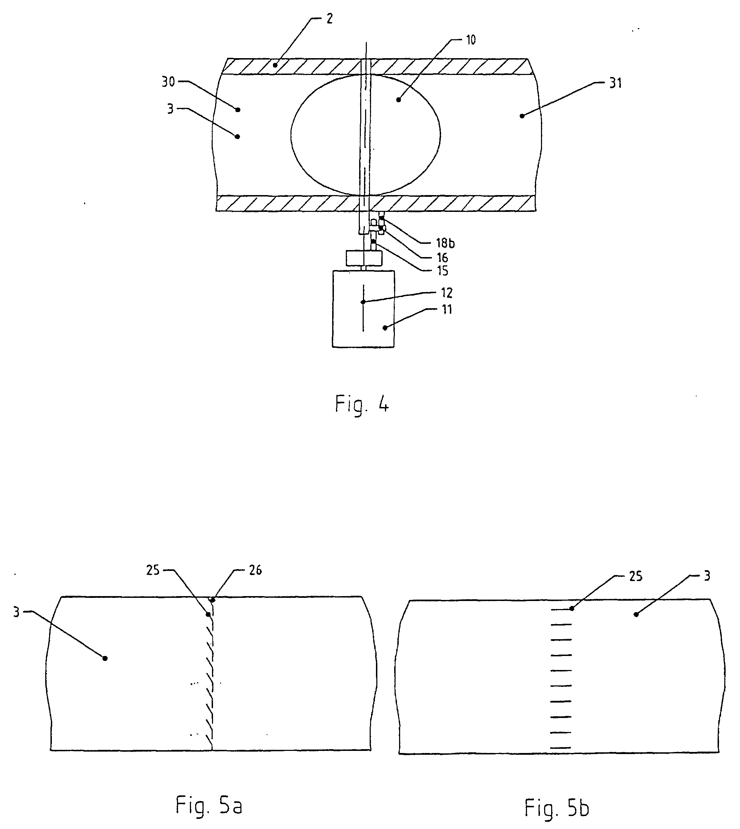

Figur 4 zeigt ein alternatives Ausführungsbeispiel einer erfindungsgemässen Vorrichtung, bei welcher die Verschlussklappe 10 elliptisch ausgebildet ist. Mit einer solchen Konstruktion erübrigt sich derjenige Anschlag, der die Verschliessstellung V definiert. Die elliptische Verschlussklappe 10 legt sich an die Innenwand des Luftdurchlasses 3 an, wodurch die Verschlussposition V definiert ist.

Figuren 5a und 5b zeigen ein weiteres, alternatives Ausführungsbeispiel einer Verschliessanordnung. Die Verschliessanordnung besteht in diesem Fall aus einer Vielzahl von Lamellen 25, welche je um eine Drehachse 26 drehbar sind. Figur 5a zeigt die Lamellen 25 in einer Verschliessstellung, in welcher der Durchlass 3 verschlossen ist. Figur 5b zeigt die geöffneten Lamellen 25, welche einen Durchfluss der Atemluft durch den Durchlass 3 erlauben. Diese Anordnung zeichnet sich insbesondere durch geringen Platzbedarf aus. Die Masse der zu bewegenden Teile liegt ausserdem nahe zur Drehachse der einzelnen Lamellen, wodurch sich ein geringeres Trägheitsmoment und ein kleinerer Aufwand an Energie zum Bewegen der Lamellenanordnung ergibt. Die einzelnen Lamellen 25 sind so untereinander verbunden, dass sie durch ein Antriebsmittel gleichzeitig bewegt werden können. Als Antriebsmittel werden wie vorangehend beschrieben lastfrei beschleunigende Antriebe verwendet.

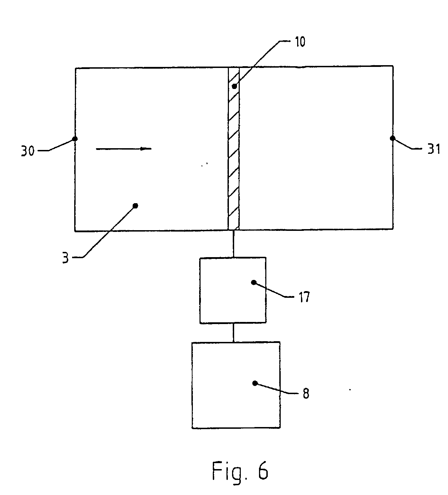

Figur 6 zeigt schematisch dem Aufbau einer erfindungsgemässen Vorrichtung. Die Verschliessanordnung 10 ist über eine Kupplungsanordnung 17 mit den Antriebsmitteln verbindbar.

Die Kupplungsanordnung kann neben der vorangehend beschriebenen Möglichkeit (Mitnehmerstift) auch aus einer Fliehkraftkupplung, spezifisch gestalteten Zahnrädern oder anderen bekannten Kupplungseinrichtungen bestehen.

## Patentansprüche

1. Vorrichtung (1) zum Messen des Atemwegwiderstandes, mit einem Gehäuse (2), welches einen Luftdurchlass (3) mit einer Einlassöffnung (30) und einer Auslassöffnung (31) aufweist,
mit Mitteln (4) zum Messen des Luftflusses durch den Luftdurchlass (3), und mit einer Verschliessanordnung (5) zum temporären Unterbrechen des Luftflusses durch Verschliessen des Luftdurchlasses (3),
sowie mit einer Messanordnung (6, 23) zum Messen des Druckes im Luftdurchlass (3),
wobei die Verschliessanordnung (5) mittels Antriebsmitteln (8) zwischen einer Ausgangsposition (A) und einer Verschlussposition (V) bewegbar ist,
**dadurch gekennzeichnet, dass** zwischen der Verschliessanordnung (5) und den Antriebsmitteln (8) eine Kupplungsanordnung (15, 16; 17) angeordnet ist, welche derart ausgelegt ist, dass eine Wirkverbindung zwischen den Antriebsmitteln (8) und der Verschliessanordnung (5) erst hergestellt wird, nachdem die Antriebsmittel (8) lastfrei und im Zeitpunkt des Anlaufens frei von einer Kopplung mit der Verschliessanordnung (5) von einer Ruheposition (R) in einen Wirkzustand (W) gebracht worden sind.

2. Vorrichtung nach Anspruch 1, **dadurch gekennzeichnet, dass** die Kupplungsanordnung (15, 16; 17) so ausgelegt ist, dass die Antriebsmittel (8) mit der Verschliessanordnung (5) erst in Eingriff treten, wenn die Antriebsmittel auf eine vorgegebene Geschwindigkeit beschleunigt wurden.

3. Vorrichtung nach einem der Ansprüche 1 oder 2, **dadurch gekennzeichnet, dass** die Verschliessanordnung (4) aus einer um eine Drehachse (13) drehbar gelagerten Verschlussklappe (10) besteht.

4. Vorrichtung nach Anspruch 3, **dadurch gekennzeichnet, dass** die Antriebsmittel (8) aus einem Motor (11) bestehen, wobei die Antriebsachse (12) des Motors koaxial zur Drehachse (13) der Verschlussklappe (10) angeordnet ist.

5. Vorrichtung nach Anspruch 4, **dadurch gekennzeichnet, dass** die Kupplungsanordnung (17) aus einem Mitnehmerstift (15) am Motor und einer exzentrischen Anschlagfläche (16) der Verschlussklappe (10) besteht, welche miteinander in Eingriff bringbar sind.

6. Vorrichtung nach Anspruch 4, **dadurch gekennzeichnet, dass** die Kupplungsanordnung (17) aus einer Fliehkraftkupplung besteht.

7. Vorrichtung nach einem der Ansprüche 1 bis 6, **dadurch gekennzeichnet, dass** der Luftdurchlass (3) mit Anschlägen (18a, 18b) versehen ist, die die Ausgangsposition (A) und die Verschlussposition (V) der Verschliessanordnung (5, 10) definieren.

8. Vorrichtung nach einem der Ansprüche 2 bis 6, **dadurch gekennzeichnet, dass** die Verschlussklappe (10) elliptisch ausgebildet ist.

9. Verfahren zum temporären Verschliessen und Öffnen eines Luftdurchlasses (3) einer Vorrichtung (1) zum Messen des Atemwegwiderstandes, **gekennzeichnet durch** folgende Schritte:
a) Lastfreies Beschleunigen von Antriebsmitteln (8) aus einer Ruheposition (R) in einen Wirkzustand (W) ohne Koppelung zwischen den Antriebsmitteln (8) und einer Verschliessanordnung (5).
b) In Eingriff bringen der Antriebsmittel (8) mit der Verschliessanordnung (5) zum Verschliessen des Luftdurchlasses (3).
c) Bewegen der Verschliessanordnung (5) von einer Ausgangsposition (A) in eine Verschlussposition (V) oder von der Verschlussposition (V) in die Ausgangsposition (A).

## Claims

1. Device (1) for measuring airway resistance, with a housing (2) that has an air passage (3) with an inlet opening (30) and an outlet opening (31),
with means (4) for measuring the air flow through the air passage (3), and with a locking arrangement (5) for the temporary interruption of the air flow by closing the air passage (3),
as well as with a measuring arrangement (6, 23) for measuring the pressure in the air passage (3),
wherein the locking arrangement (5) can be moved between a starting position (A) and a closed position (V) by means of drive elements (8),
**characterised in that**
arranged between the locking arrangement (5) and the drive elements (8) is a coupling arrangement (15, 16; 17), which is designed such that a working connection between the drive elements (8) and the locking arrangement (5) is produced only after the drive elements (8) have been brought - load-free and, at the time of starting, free from any coupling with the locking arrangement (5) - from a resting position (R) into a working state (W).

2. Device in accordance with claim 1, **characterised in that** the coupling arrangement (15, 16; 17) is designed such that the drive elements (8) engage with the locking arrangement (5) only when the drive elements have been accelerated to a predetermined speed.

3. Device in accordance with one of the claims 1 or 2, **characterised in that** the locking arrangement (4) comprises a sealing cap (10) that is pivoted around a swivel axis (13).

4. Device in accordance with claim 3, **characterised in that** the drive elements (8) comprise a motor (11), wherein the drive axis (12) of the motor is arranged coaxially to the swivel axis ( 13) of the sealing cap (10).

5. Device in accordance with claim 4, **characterised in that** the coupling arrangement (17) comprises a drive pin (15) on the motor and an eccentric stopping face (16) of the sealing cap (10), which can be brought to engage with one another.

6. Device in accordance with claim 4, **characterised in that** the coupling arrangement (17) comprises a centrifugal clutch.

7. Device in accordance with one of the claims 1 to 6, **characterised in that** the air passage (3) is equipped with limit stops (18a, 18b) which define the starting position (A) and the closed position (V) of the locking arrangement (5, 10).

8. Device in accordance with one of the claims 2 to 6, **characterised in that** the sealing cap (10) is designed elliptically.

9. Method for the temporary closing and opening of an air passage (3) of a device (1) for measuring airway resistance, **characterised by** the following steps:
a) Load-free acceleration of drive elements (8) from a resting position (R) into a working state (W), without a coupling between the drive elements (8) and a locking arrangement (5).
b) Engagement of the drive elements (8) with the locking arrangement (5) for closing the air passage (3).
c) Movement of the locking arrangement (5) from a starting position (A) into a closed position (V) or from the closed position (V) into the starting position (A).

## Revendications

1. Dispositif (1) pour mesurer la résistance des voies respiratoires, comportant
un boîtier (2) qui présente un passage d'air (3) avec une ouverture d'entrée (30) et une ouverture de sortie (31) ,
des moyens (4) pour mesurer l'écoulement d'air dans le passage (3), avec un obturateur (5) pour interrompre temporairement l'écoulement d'air grâce à une obturation dudit passage (3),
et un dispositif de mesure (6, 23) pour mesurer la pression dans le passage (3),
l'obturateur (5) étant mobile grâce à des moyens d'entraînement (8) entre une position de départ (A) et une position fermée (V),
**caractérisé en ce qu'**il est prévu entre l'obturateur (5) et les moyens d'entraînement (8) un dispositif d'accouplement (15, 16 ; 17) qui est monté de telle sorte qu'une liaison fonctionnelle entre les moyens d'entraînement (8) et l'obturateur (5) n'est réalisée qu'une fois que les moyens d'entraînement (8) ont été amenés, sans contrainte et sans accouplement à l'obturateur (5) au moment du démarrage, d'une position de repos (R) à une position active (W).

2. Dispositif selon la revendication 1, **caractérisé en ce que** le dispositif d'accouplement (15, 16 ; 17) est conçu pour que les moyens d'entraînement (8) ne viennent en prise avec l'obturateur (5) qu'après avoir été accélérés jusqu'à une vitesse prédéfinie.

3. Dispositif selon la revendication 1 ou 2, **caractérisé en ce que** l'obturateur (5) se compose d'un clapet (10) monté pivotant autour d'un axe de rotation (13).

4. Dispositif selon la revendication 3, **caractérisé en ce que** les moyens d'entraînement (8) se composent d'un moteur (11), l'axe d'entraînement (12) du moteur étant coaxial par rapport à l'axe de rotation (13) du clapet (10).

5. Dispositif selon la revendication 4, **caractérisé en ce que** le dispositif d'accouplement (17) se compose d'une tige d'entraînement (15) prévue sur le moteur et d'une surface de butée excentrique (16) du clapet (10), qui sont aptes à être mises en contact.

6. Dispositif selon la revendication 4, **caractérisé en ce que** le dispositif d'accouplement (17) se compose d'un accouplement centrifuge.

7. Dispositif selon l'une des revendications 1 à 6, **caractérisé en ce que** le passage d'air (3) est pourvu de butées (18a, 18b) qui définissent la position de départ (A) et la position fermée (V) de l'obturateur (5, 10).

8. Dispositif selon l'une des revendications 2 à 6, **caractérisé en ce que** le clapet (10) a une forme elliptique.

9. Procédé pour fermer et ouvrir temporairement un passage d'air (3) d'un dispositif (1) pour mesurer la résistance des voies respiratoires, **caractérisé par** les étapes suivantes :
a) accélération, sans contrainte, des moyens d'entraînement (8) d'une position de repos (R) à une position active (W), sans accouplement entre les moyens d'entraînement (8) et un obturateur (5),
b) mise en contact des moyens d'entraînement (8) et de l'obturateur (5) en vue de fermer le passage d'air (3),
c) déplacement de l'obturateur (5) d'une position de départ (A) à une position fermée (V) ou de la position fermée (V) à la position de départ (A).
